Europäisches Patentamt

European Patent Office

Office Européen des brevets

(19)

(11) Veröffentlichungsnummer : **0 243 593 B1**

(12) **EUROPÄISCHE PATENTSCHRIFT**

(45) Veröffentlichungstag der Patentschrift :
02.01.91 Patentblatt 91/01

(51) Int. Cl.⁵ : **A61F 2/10**

(21) Anmeldenummer : **87102217.4**

(22) Anmeldetag : **17.02.87**

(54) **Verfahren und Vorrichtung zum Anbringen von Haaren an Hautabschnitten.**

(30) Priorität : 29.04.86 DE 3614452

(43) Veröffentlichungstag der Anmeldung :
04.11.87 Patentblatt 87/45

(45) Bekanntmachung des Hinweises auf die
Patenterteilung :
02.01.91 Patentblatt 91/01

(84) Benannte Vertragsstaaten :
AT BE CH DE ES FR GB GR IT LI LU NL SE

(56) Entgegenhaltungen :
FR-A- 1 587 858

(73) Patentinhaber : Czech, Joachim
Jahnstrasse19
D-8405 Donaustauf (DE)

(72) Erfinder : Czech, Joachim
Jahnstrasse19
D-8405 Donaustauf (DE)

(74) Vertreter : Patentanwälte Grünecker,
Kinkeldey, Stockmair & Partner
Maximilianstrasse 58
D-8000 München 22 (DE)

## Beschreibung

Die vorliegende Erfindung bezieht sich auf ein Verfahren zum Anbringen von Haaren an Hautabschnitten, die gegebenenfalls Resthaar aufweisen können, insbesondere für die Verbesserung oder Wiederherstellung eines Haarbesatzes der Kopfhaut. Die Erfindung bezieht sich ferner auf eine Vorrichtung zur Durchführung dieses Verfahrens.

Zur Bekämpfung des insbesondere bei Männern auftretenden Haarausfalls sowie zur Förderung des erneuten Haarwuchses nach erfolgter Glatzenbildung sind eine Vielzahl kosmetischer Präparate bekannt, die Wirksamkeit dieser Mittel ist jedoch im allgemeinen gering geblieben. Dies betrifft insbesondere alle Versuche, ein erneutes Haarwachstum an denjenigen Stellen der Kopfhaut anzuregen, die bereits von einem völligen Haarausfall betroffen sind.

Die Benutzung von Toupets, Haarteilen und Perücken wirft eine Vielzahl anwendungstechnischer und ästhetischer Probleme auf, die vielfach zu einer Verunsicherung des betroffenen Personenkreises führen.

Es wurden ferner bereits Haartransplantationen im Kopfhaar ausgeführt, bei denen Kopfhaar zusammen mit dem zugehörigen Hautabschnitt aus einem Bereich nicht oder wenig geschädigten Haarwuchses in ein vom Haarausfall betroffenes Gebiet der Kopfhaut verpflanzt wurde. Es hat sich jedoch gezeigt, daß auch die verpflanzten, zunächst noch eingewurzelten Haare ausfielen, mit der Folge nicht nur des transplantationsbedingten Haarverlustes in ungeschädigten Bereichen, sondern auch zusätzlicher, auffälliger Narbenbildungen auf den vom Haarausfall betroffenen Flächen.

Der Erfindung liegt daher die Aufgabe zugrunde, ein Verfahren der eingangs genannten Art anzugeben, mit dem es auf unkomplizierte Weise möglich ist, verlorengegangenen Haarschmuck unter Wahrung eines völlig natürlichen Ercheinungsbildes, insbesondere im Bereich des Haaransatzes, unverlierbar zu ersetzen.

Der Erfindung liegt ferner die Aufgabe zugrunde, eine Vorrichtung zu schaffen, die die Ausführung des obigen Verfahrens auf unkomplizierte Weise und unter äußerst präsiser Lokalisierung des Applikationsortes bei minimaler Belastung der dem Verfahren unterworfenen Person gestattet.

Vorzugsweise soll daher das Verfahren in Einrichtungen des Dienstleistungsgewerbes ausführbar sein.

Diese Aufgaben werden erfindungsgemäße entsprechend den kennzeichnenden Merkmalen der Ansprüche 1 und 8 gelöst.

Demzufolge besteht das Verfahren entweder darin, Fremdhaar mit einem Ansatzende im Ansatzpunkt in Kontakt mit dem gewünschten Hautabschnitt zu bringen und anschließend das Fremdhaar mittels eines Laserstrahls an der zugehörigen Hautpartie anzuschmelzen oder bei Vorhandensein von Resthaar das Ansatzende des Fremdhaares unmittelbar an dem Fußpunkt des Resthaares auf den Hautabschnitt mit diesem Hautabschnitt in Kontakt zu bringen und in diesem Bereich das Fremdhaar nahe des Hautabschnitts an das Resthaar anzuschmelzen.

Vorzugsweise wird das Fremdhaar zu einzelnen Haaren vereinzelt und jedes Haar mit seinem Ansatzende in einem Ansatzpunkt, auf dem der Laserstrahl gerichtet ist, in Kontakt mit der Hautpartie, vorzugsweise mit der Kopfhaut gebracht.

Mittels des Laserstrahls kann auf einer punktförmigen Fläche, dem Ansatzpunkt für das Fremdhaar, sehr schnell eine sehr hohe Energiedichte erzielt werden, die zu einer kurzzeitigen Flexibilisierung der oberen Hautschichten, verbunden mit einer Schmelzerweichung der Haarspitzen im Bereich des Ansatzendes eines Haares oder Haarbüschels mit dem Ergebnis führt, daß der Fremdhaarkörper sich innig fest mit dem zugeordneten Hautbereich anschmelzend verbindet. Zur genauen Ausrichtung und Lageorientierung des Ansatzendes des Fremdhaares und zur Ausübung einer entsprechenden Kontrolle ist es vorteilhaft, wenn der Ansatzpunkt, d.h. Zielpunkt des Laserstrahls auf der Haut vor dem Anschmelzen des Fremdhaares optisch markiert wird.

Bei noch vorhandenem, jedoch ungenügend entwickeltem, Resthaar kann dieses vorzugsweise unmittelbar an der Stelle seiner Einwuurzelung in der Kopfhaut als "Anknüpfungspunkt" für die Festlegung eines neuen, aus Fremdhaar separierten "Ersatzhaares" benutzt werden, mit der Folge, daß die örtliche Wärmebelastung der Kopfhaut drastisch reduziert wird. In diesem Fall wird das neue Haar direkt mit dem Resthaar möglichst dicht an der Kopfhaut durch den Laserstrahl verschmolzen.

Weitere vorteilhafte Ausgestaltungen des geschilderten Verfahrens sind in den weiteren Unteransprüchen dargelegt.

Zur Durchführung des Verfahrens umfaßt die erfindungsgemäße Vorrichtung einen mobilen Einstzkopf, in dem eine Laserstrahlquelle angeordnet sowie eine Führungseinrichtung für das Fremdhaar vorgesehen ist, wobei das Fremdhaar durch eine Applikationsöffnung des Einsatzkopfes mit seinem Ansatzende im Ansatzpunkt in Kontakt mit dem zugehörigen Hautabschnitt bringbar ist und der von der Laserstrahlquelle emittierte Laserstrahl in diesem Ansatzpunkt auf den Hautabschnitt trifft.

Gegebenenfalls wird der Ansatzpunkt durch den Fußpunkt eines in dem jeweiligen Hauptabschnitt noch vorhandenen Resthaares festgelegt und das Fremdhaar direkt mit dem Resthaar selbst nahe des Hautabschnitts verschmolzen.

Vorzugsweise ist auch eine optische Markierungseinrichtung vorgesehen, die es gestattet, eine genaue Lagezuordnung des Ansatzendes des Haares bzw. Haarbüschels zum Ansatzpunkt vorzunehmen und zugleich eine optische Kontrolle bei der Durchführung des Verfahrens ermöglicht.

Weitere vorteilhafte Ausgestaltungen der Vorrichtung zur Durchführung des erfindungsgemäßen Verfahrens sind in den übrigen Unteransprüchen dargelegt.

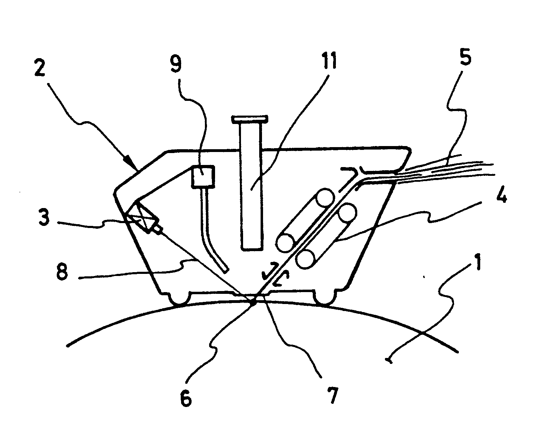

Die Erfindung wird nachstehend anhand eines Ausführungsbeipsiels und einer zugehörigen Zeichnung näher erläutert, wobei die Zeichnung schematisch einen Einsatzkopf im Querschnitt zeigt.

Zur unverlierbaren Anbringung von Fremdhaar an einer Kopfhaut 1, die sich durch Glatzenbildung auszeichnet, wird ein Einsatzkopf 2 verwendet, dessen funktionale Grundbestandteile eine Laserstrahlquelle 3 sowie eine Zuführungsvorrichtung 4 sind, wobei die Zuführungsvorrichtung 4 zum Zuführen und Ausrichten von natürlichem Fremdhaar 5, das gegebenenfalls zu Haarbüscheln zusammengefaßt wird, dient. Im allgemeinen wird jedoch vor der Applikation das Fremdhaar 5 in einzelne Haare 5 separiert und jedes Haar 5 getrennt voneinander mit einem vorangeschobenen Ansatzende durch eine Applikationsöffnung 7 an der Unterseite des Einsatzkopfes 2 um ein geringes Maß hervorgeschoben, wobei ein Ansatzpunkt 6, d.h. der Berührungspunkt des Haares 5 mit der Kopfhaut 1 zugleich den Zielpunkt eines von der Laserstrahlquelle 3 emittierten Laserstrahls 8 auf der Kopfhaut 1 bildet.

Unter "Fremdhaar" im Sinne dieser Anmeldung wird natürliches Haar fremder Personen, aber auch der eigenen Person verstanden, deren Haardichte erfindungsgemäß verbessert werden soll. D.h. es liegt durchaus im Rahmen dieser Anmeldung, körpereigenes Haar als "Fremdhaar", z.B. abgeschnitten von Bereichen mit ungeschädigtem Kopfhaarwuchs, zu verwenden.

Der Ansatzpunkt 6 kann unabhängig von dem Laserstrahl 8 auch durch eine optische Markierungseinrichtung 9 markiert werden, die zu diesem Zweck in Verbindung mit einer Beleuchtungsquelle, vorzugsweise ein Lichtleitkabel 10, verwendet. Selbstverständlich kann hierfür auch ein Blendenrohr angewandt werden, das auf der Kopfhaut 1 einen Lichtfleck zur Markierung des Ansatzpunktes erzeugt, der etwa der Größe des von dem Laserstrahl 8 getroffenen Bereichs der Kopfhaut 1 entspricht.

Vorzugsweise wird die Laserstrahlquelle 3 als $CO_2$-Laser ausgeführt, um unerwünschte Beeinflussungen tiefer liegender Hautpartien zu vermeiden.

Ferner ist, gegbenenfalls unter Einsatz einer Vergrößerungseinrichtung, ein Beobachtungstubus 11 vorgesehen, der eine Beobachtung des Gebietes ermöglicht, das durch den Ansatzpunkt 6 bestimmt ist, in dem das Ansatzende des Fremdhaarbüschels 5 sowie der von der Laserstrahlquelle 3 emittierte Laserstrahl 8 auf der Kopfhaut 1 auftreffen. Auf diese Weise kann sowohl die Vorbereitung des Verahrens der Haarbefestigung als eine Anschmelzung des Haares 5 selbst durch den Laserstrahl 8 kontrolliert werden. Vorzugsweise ist der Beobachtungstubus 11 als Mikroskop ausgeführt.

Die Führungseinrichtung 4 ermöglicht auch eine Lagefixierung des Haares 5 sowie eine Lösung dieser Lagefixierung nach erfolgter Schmelzbefestigung des Haares 5 an der Kopfhaut 1. Ferner ist eine hier nicht gezeigte, handbetätigbare Korrekturvorrichtung für die Zuführungsvorrichtung vorgesehen, um jederzeit eine zielgenaue, auf den Ansatzpunkt orientierte Lageeinstellung der Fremdhaarzuführung vornehmen zu können.

Dies ist insbesondere dann erforderlich, wenn zur Verbesserung der Anpaßbarkeit des Einsatzkopfes 2 an den jeweiligen Abschnitt der Kopfhaut 1 eine, die Applikationsöffnung 7 tragende Bodenwandung des Gehäuses des Einsatzkopfes 2 flexibel ausgeführt ist oder der Einsatzkopf 2 an seiner Unterseite elastische Auflagekörper aufweist. Zur Förderung einer Verschieblichkeit des Einsatzkopfes 2 entlang der Kopfhaut 1 kann dieser auch an seiner Unterseite Bewegungswalzen oder Rollen aufweisen, durch die zugleich der Abstand der Applikationsöffnung 7 und der Kopfhaut 1 festgelegt werden kann. Falls erforderlich, kann zur Vergrößerung der Grundhelligkeit des durch den Beobachtungstubus 11 vermittelten Bildes noch eine weitere Beleuchtungseinrichtung geringer Leistung innerhalb des Einsatzkopfes 2 angeordnet sein, die in der schematischen Darstellung des Einsatzkopfes 2 nicht gezeigt ist. Der Einsatzkopf zur Anwendung des oben dargelegten Verfahrens arbeitet wie folgt :

Nach dem Aufsetzen des Einsatzkopfes auf den betreffenden Abschnitt der Kopfhaut 1 wird zunächst der Ansatzpunkt durch die optische Einrichtung 9 markiert sowie Fremdhaar 5 der gewünschten Länge und Farbe durch die Führungseinrichtung 4 mit dem Ansatzende bis zur Berührung an die Kopfhaut 1 herangeführt. Mit Hilfe des Beobachtungstubus 11 kann nunmehr eine Korrektur der Lage des zugeführten Fremdhaares 5 derart erfolgen, daß das Ansatzende eines Haares 5 oder eines Haarbüschels exakt die Kopfhaut im durch die Markierungseinrichtung 9 indizierten Ansatzpunkt 6 berührt. Anschließend wird die Laserstrahlquelle 3 aktiviert und durch den energiereichen Laserstrahl 8 kurzzeitig eine hohe Energiedichte, d.h. eine hohe Wärmedichte im Ansatzpunkt 6, d.h. im Verbindungsbereich zwischen der Kopfhaut 1 und dem Haar 5 realisiert. Dies führt sowohl zu einer lokalen Flexibilisierung der Kopfhaut als auch zu Schmelzerweichung des Ansatzendes des Haares 5 mit der Folge des Anschmelzens dieses Ansatzendes an der Kopfhaut 1, so daß nach Beendigung der kurzzeitigen Laserstrahlanwendung das Fremdhaar 5 innig und unverlierbar mit der Kopfhaut 1 verbunden ist.

Anschließend wird der gleiche Vorgang an einem benachbarten Ansatzpunkt unter Zuführung eines weiteren Haares 5 wiederholt, so daß Fremdhaare in sehr dichter Anordnung mit der Kopfhaut 1 verbunden werden können.

Die Arbeitsweise des Einsatzkopfes 2 kann auf vielfältige Weise modifiziert werden, z.B. durch eine Verbreiterung der Führungseinrichtung 4 derart, daß diese selbst ein Bündel von zugeführtem Fremdhaar zu einzelnen, benachbarten Haaren oder Haarbüscheln vereinzelt und diese an der Unterseite des Einsatzkopfes 2 in einer dichten Reihung der zugehörigen Ansatzenden bereithält. Bei entsprechender Zuordnung der Zuführungsvorrichtung zu einer Bewegungsbahn der Laserstrahlquelle 3 kann diese auch beweglich angeordnet werden und, nach optischer Kontrolle zumindest eines der Ansatzpunkte 6, durch entsprechende Relativbewegung des Laserstrahls 3 zu den von der Führungseinrichtung 4 bereitgehaltenen Fremdhaarbüschel in Haaren 5 mit der Kopfhaut 1 verschmolzen werden.

Zur Förderung des Anschmelzvorgangs können zusätzlich zum Ansatzpunkt 6 Hilfsstoffe, vorzugsweise Polyvinylpyrrolidone, zugeführt werden.

Es ist selbstverständlich auch möglich, die Führungseinrichtung 4, die trichterartige Mittel zum Verdichten des Ansatzendes von Fremdhaarbüscheln bzw. zum Vereinzeln von Haaren 5 aufweisen kann, fest anzuordnen, so das vorgeschobene, durch die Applikationsöffnung 7 tretende Ansatzende des Haares 5 den Ansatzpunkt 6 bestimmt. In diesem Fall ist die Markierungseinrichtung 9, die fest mit der Laserstrahlquelle 3 gekoppelt ist, gemeinsam mit dieser beweglich. Unter Beobachtung wird die Markierungseinrichtung 9 und damit gleichzeitig die Laserstrahlquelle 3 auf den nunmehr durch das Ansatzende des Haares 5 bestimmten Ansatzpunkt 6 ausgerichtet, anschließend erfolgt die bereits beschriebene Anschmelzung des Ansatzendes des Haares 5 im Ansatzpunkt 6 durch den Laserstrahl 8. Durch die jeweilige Ausrichtung der Laserstrahlquelle 3 oder der Führungseinrichtung 4 kann jedwede, durch die Kopfkrümmung bedingte mögliche Abstandsvariation zwischen dem Einsatzkopf 2 bzw. dessen Applikationsöffnung 7 und der Kopfhaut 1 ausgeglichen werden, so daß der Schnittpunkt von Laserstrahl 8 und Zuführungsebene des Haares 5 stets genau in der den Ansatzpunkt 6 enthaltenden Kopfhautebene liegt.

Es kann auch wünschenswert sein, das Fremdhaar 5 möglichst senkrecht auf die Kopfhaut 1 zu führen. In diesem Fall kann die Führungseinrichtung 4 einen vertikalen Einführungstrichter bilden und in der vorliegenden Zeichnung mit dem Beobachtungstubus die Plätze tauschen.

Das neue Verfahren sowie die hierzu vorgesehene Vorrichtung in Gestalt des Einsetzkopfes 2 sind außerordentlich einfach ausführbar, so daß eine Anwendung des Einsatzkopfes 2 im kosmetischen Dienstleistungsgewerbe erfolgen kann. Aufgrund der hohen Energiedichte im Ansatzpunkt kann ein praktisch augenblickliches Anschmelzen des Fremdhaares an die Kopfhaut erfolgen, so daß keine nennenswerte Belastung des Behandelten auftritt. Bezüglich des Vereinzelungsgrads der angeschmolzenen Haare sowie der Verteilungsdichte besteht ein großer Anwendungsspielraum.

Es kann vorteilhaft sein, zum Anschmelzen möglichst "starkes" Haar mit relativ großem Haardurchmesser zu verwenden, wie es z.B. bei fernöstlichen oder mittel- und südamerikanischen Bevölkerungsgruppen angetroffen wird.

Die Erfindung wurde bisher anhand eines Ausführungsbeispiels erläutert, bei dem Fremdhaar als Ersatzhaar unmittelbar an der Kopfhaut angeschmolzen wurde. Eine Laserstrahlanschmelzung von neuen Haaren 5 kann jedoch nach einem weiteren Aspekt der vorliegenden Lösung auch unter Verwendung von Resthaaren auf der Kopfhaut 1 der jeweiligen Person erfolgen. Einzelne Resthaare, die zum Teil in ihrem natürlichen Wachstum ebenfalls geschädigt sind, werden vielfach auch in Bereichen der Kopfhaut 1 noch angetroffen, die im wesentlichen keinen Haarbesatz mehr zeigen. Diese Resthaare sind, gegebenenfalls auf ein kurzes Reststück gekürzt, in besonders vorteilhafter Weise als Basis für das Anschmelzen der neuen Haare 5 verwendbar.

In diesem Fall wird ein Fremhaar 5 unmittelbar im "Fußpunkt" des Resthaares, in dem dieses aus der Kopfhaut 1 heraustritt, an die Kopfhaut 1 angesetzt, so daß das neue Haar 5 einerseits die Kopfhaut 1 und andererseits das Resthaar unmittelbar in seinem Verbindungspunkt mit der Kopfhaut 1 berührt. Das Anschmelzen durch den Laserstrahl 8 erfolgt in diesem Falle jedoch geringfügig oberhalb der Kopfhaut 1 unmittelbar durch eine punktförmige Laserstrahlverschmelzung zwischen dem neuen Haar 5 und dem Resthaar. Der Anschmelzpunkt soll so dicht wie möglich an der Kopfhaut 1 liegen. Gegebenenfalls kann die Anschmelzung sowohl partiell an dem Resthaar als auch an der Kopfhaut 1 erfolgen.

Die mögliche Einbeziehung von Resthaar hat die großen Vorteile einer Verschmelzung strukturgleichen Materials verbunden mit völliger Verminderung selbst mikroskopischer Kopfhautveränderungen, so daß die Belastung der Person, an deren Kopfhaut die Haare angebracht werden, in Bereichen mit Resthaaren weiter absinkt.

Durch die Erfindung wird es möglich, die nachteiligen Folgen der bisher nicht ursächlich behandelbaren Glatzenbildung zumindest drastisch zu reduzieren und einen Haarbesatz zu ermöglichen, der erstmals dem Erscheinungsbild des ursprünglichen Haarwuchses angenähert werden kann.

## Ansprüche

1. Verfahren zum Anbringen von Haaren an, gegebenenfalls Resthaar aufweisenden, Hautabschnitten, insbesondere für eine Verbesserung oder Wiederherstellung eines Haarbesatzes der Kopfhaut, dadurch gekennzeichnet, daß Fremdhaar (5) mit einem Ansatzende in einem Ansatzpunkt (6) in Kontakt mit dem Hautabschnitt (1) gebracht und anschließend mittels eines Laserstrahls (8) im Bereich des Ansatzpunktes (6) an dem Hautabschnitt (1) und/oder dem Resthaar angeschmolzen wird.

2. Verfahren nach Anspruch 1, dadurch gekennzeichnet, daß das Fremdhaar in separierten, einzelnen Haaren (5) an einer Kopfhaut (1) angeschmolzen wird.

3. Verfahren nach Anspruch 1, dadurch gekennzeichnet, daß bei einer verhältnismäßig kurze Resthaare aufweisenden Kopfhaut (1) die Fußpunkte der Resthaare auf der Kopfhaut (1) jeweils den Ansatzpunkt (6) festlegen, an dem das Ansatzende eines verhältnismäßig langen Haares (5) in Kontakt mit der Kopfhaut gebracht wird und anschließend das Haar (5) an das Resthaar nahe des Ansatzpunktes (6) aangeschmolzen wird.

4. Verfahren nach Anspruch 1, dadurch gekennzeichnet, daß Fremdhaar zu Fremdhaarbüscheln zusammengefaßt und jedes Fremdhaarbüschel an dem Ansatzende verdichtet und in einem Ansatzpunkt (6) in Kontakt mit dem Hautabschnitt einer Kopfhaut (1) gebracht wird, und der Laserstrahl (8) in dem Ansatzpunkt (6) auf die Kopfhaut (1) trifft.

5. Verfahren nach Anspruch 1, dadurch gekennzeichnet, daß das Fremdhaar in Haarbüscheln zugeführt, vor dem Anschmelzen zu einzelnen Haaren vereinzelt und anschließend jedes Haar separat angeschmolzen wird.

6. Verfahren nach Anspruch 1, dadurch gekennzeichnet, daß der Ansatzpunkt (6) vor dem Anschmelzen optisch markiert wird.

7. Verfahren nach Anspruch 2, dadurch gekennzeichnet, daß die Orientierung des Laserstrahls (8) den Ansatzpunkt (6) des Haares (5) bestimmt und das Haar (5) festgehalten wird, wenn sich dessen Ansatzende im Ansatzpunkt (6) befindet.

8. Verfahren nach Anspruch 2 oder 3, dadurch gekennzeichnet, daß die Lagezuordnung des Haares (5) zu dem Ansatzpunkt (6) beobachtet wird.

9. Verfahren nach Anspruch 2 oder 3, dadurch gekennzeichnet, daß der Laserstrahl (8) bewegbar geführt wird und eine Bewegungsbahn des Laserstrahls (8) auf der Kopfhaut (1) eine Mehrzahl von Ansatzpunkten (6) miteinander verbindet, in denen das jeweilige Ansatzende (6) von Haaren (5) oder Fremdhaarbüscheln festliegt.

10. Verfahren nach Anspruch 2, dadurch gekennzeichnet, daß eine Mehrzahl von Haaren (5) mit ihren Ansatzenden in Kontakt mit der Kopfhaut (1) steht und nacheinander unter Änderung der Richtung des Laserstrahls (8) an die Kopfhaut (1) angeschmolzen wird.

11. Verfahren nach Anspruch 3, dadurch gekennzeichnet, daß eine Mehrzahl von Haaren (5) mit ihren Ansatzenden in einzelnem Kontakt mit der Kopfhaut (1) und je einem zugehörigen Resthaar steht und nacheinander unter Änderung der Richtung des Laserstrahls (8) im Bereich des Ansatzpunktes (6) an das Resthaar angeschmolzen werden.

12. Vorrichtung zur Durchführung des Verfahrens nach Anspruch 1, dadurch gekennzeichnet, daß in einem mobilen Einsatzkopf (2) eine Laserstrahlquelle (3) angeordnet sowie eine Führungseinrichtung (4) für Fremdhaar (5) vorgesehen ist, derart, daß das Fremdhaar (5) mit einem Ansatzende voran durch eine Applikationsöffnung (7) führbar ist und ein Ansatzpunkt (6) des Fremdhaares (5) durch Kontakt des Ansatzendes des Fremdhaares (5) mit einem Hautabschnitt (1) im Schnittpunkt mit einem von der Laserstrahlquelle (3) emittierten Laserstrahl (8), gegebenenfalls in praktischer Koinzidenz mit einem Fußpunkt eines Resthaares auf dem Hautabschnitt (1) bestimmt ist.

13. Vorrichtung nach Anspruch 12, dadurch gekennzeichnet, daß der Einsatzkopf (2) eine optische Markierungseinrichtung (9) mit einem Lichtleitkabel (10) aufweist.

14. Vorrichtung nach Anspruch 12, dadurch gekennzeichnet, daß der Einsatzkopf (2) eine optische Beobachtungseinrichtung (11), insbesondere im Mikroskop, aufweist.

15. Vorrichtung nach Anspruch 12, dadurch gekennzeichnet, daß die Führungseinrichtung (4) eine Zuführungs-, Zentrier- und Festhaltevorrichtung umfaßt.

16. Vorrichtung nach Anspruch 15, dadurch gekennzeichnet, daß die Führungseinrichtung (4) Transportwalzen umfaßt.

17. Vorrichtung nach Anspruch 16, dadurch gekennzeichnet, daß die Transportwalzen die Formierung einzelner Haare (5) aus einem Fremdhaarbündel unterstützt.

18. Vorrichtung nach Anspruch 15, dadurch gekennzeichnet, daß die Führungseinrichtung Fremdhaarbüschel aufnimmt, aus denen in der Zentriervorrichtung einzelne Haare (5) vereinzelbar sind.

19. Vorrichtung nach Anspruch 15, dadurch gekennzeichnet, daß die Führungseinrichtung die Formierung einzelner Fremdhaarbüschel aus einem Fremdhaarbüschel unterstützt.

20. Vorrichtung nach Anspruch 12, dadurch gekennzeichnet, daß durch die Führungseinrichtung (4) eine Mehrzahl von Haaren (5) zum jeweiligen Applikationsort zugeführt und die Laserstrahlquelle (3) relativ zur Führungseinrichtung (4) bewegbar ist.

21. Vorrichtung nach Anspruch 12, dadurch gekennzeichnet, daß der Einsatzkopf (2) ein Gehäuse aus Kunststoff aufweist, dessen Bodenwandung flexibel ausgeführt ist oder elastische Abstützkörper trägt.

22. Vorrichtung nach Anspruch 21, <u>dadurch gekennzeichnet</u>, daß die Bodenwandung zumindest nahe der Applikationsöffnung (7) durch Bewegungskörper in einem definierten Abstand zu dem Hautabschnitt gehalten (1) ist.


**Claims**

1. A method of attaching hair to portions of skin, which may have residual hair, more particularly for improving or restoring the hair on the scalp, characterised in that an attachment end of foreign hair (5) is brought into contact with a skin portion (1) at a point (6) of attachment and is then fused to the skin portion (1) and/or the residual hair by a laser beam (8) in the region of the point (6) of attachment.

2. A method according to claim 1, characterised in that separated individual foreign hairs (5) are fused to a scalp (1).

3. A method according to claim 1, characterised in that in the case of a scalp (1) having relatively short residual hair, the bases of the residual hairs (1) on the scalp (1) determine the respective attachment points (6) at which the attachment end of a relatively long hair (5) is brought into contact with the scalp and the hair (5) is subsequently fused to the residual hair near the attachment point (6).

4. A method according to claim 1, characterised in that foreign hair is collected in tufts and each tuft is compressed at the attachment end and brought into contact with the skin portion of a scalp (1) at an attachment point (6), and the laser beam (8) strikes the scalp (1) at the attachment point (6).

5. A method according to claim 1, characterised in that the foreign hair is supplied in tufts and separated into individual hairs before fusing, after which each hair is separately fused.

6. A method according to claim 1, characterised in that the attachment point (6) is optically marked before fusing.

7. A method according to claim 2, characterised in that the orientation of the laser beam (8) determines the attachment point (6) of the hair (5) and the hair (5) is secured when its attachment end is at the attachment point (6).

8. A method according to ciaim 2 or 3, characterised in that the positioning of the hair (5) at the attachment point (6) is observed.

9. A method according to claim 2 or 3, characterised in that the laser beam (8) is movably guided and a track of the laser beam (8) on the scalp (1) connects a plurality of attachment points (6) in which the respective attachment ends (6) of hairs (5) or foreign hair tufts are secured.

10. A method according to claim 2, characterised in that the attachment ends of a plurality of hairs (5) are in contact with the scalp (1) and are successively fused to the scalp (1) while altering the direction of the laser beam (8).

11. A method according to claim 3, characterised in that the attachment ends of a plurality of hairs (5) are brought individually into contact with the scalp (1) and with respective associated residual hairs and are successively fused to the residual hair in the region of the attachment point (6), while altering the direction of the laser beam (8).

12. A device for working the method according to claim 1, characterised in that a laser-beam source (3) is disposed in a mobile operational head (2) and a device (4) for guiding foreign hair (5) is provided so that an attachment end of the foreign hair (5) can be guided through an application opening (7) and the point (6) for attaching the foreign hair (5) is determined by contact of the attachment end of the foreign hair (5) with a skin portion (1) at the point of intersection with a laser beam (8) emitted by the source (3), substantially coinciding if required with a base of a residual hair on the skin portion.

13. A device according to claim 12, characterised in that the operational head (2) comprises an optical marking device (9) and an optical fibre cable (10).

14. A device according to claim 12, characterised in that the operational head (2) comprises an optical observation device (11), more particularly in a microscope.

15. A device according to claim 12, characterised in that the guide device (4) comprises a supply, centring and retaining device.

16. A device according to claim 15, characterised in that the guide device (4) comprises conveying rollers.

17. A device according to claim 16, characterised in that the conveying rollers assist the process of forming individual hairs (5) out of a tuft of foreign hair.

18. A device according to claim 15, characterised in that the guide device takes up tufts of foreign hair, out of which individual hairs (5) can be separated in the centring device.

19. A device according to claim 15, characterised in that the guide device assists the process of forming individual foreign-hair tufts out of a tuft

20. A device according to claim 12, characterised in that the guide device (4) supplies a plurality of hairs (5) to the respective place of application and the laser-beam source (3) is movable relative to the guide device.

21. A device according to claim 12, characterised in that the operational head (2) has a plastics casing, the bottom wall of which is flexible or bears resilient abutment members.

22. A device according to claim 21, characterised in that the bottom wall, at least near the application opening (7), is held by moving members at a defined distance from the scalp portion.

## Revendications

1. Procédé de fixation de cheveux sur des parties du cuir chevelu présentant le cas échéant des cheveux résiduels, en particulier pour une amélioration ou une reconstitution d'une chevelure sur le cuir chevelu, caractérisé en ce que le cheveu étranger (5) est mis en contact par une extrémité de fixation, à un point de fixation (6), avec la partie du cuir chevelu (1) et est ensuite fondu à l'aide d'un rayon laser (8), dans la zone du point de fixation (6), sur la partie du cuir chevelu (1) et/ou le cheveu résiduel.

2. Procédé selon la revendication 1, caractérisé en ce que le cheveu étranger est fondu sur un cuir chevelu (1) en différents cheveux séparés (5).

3. Procédé selon la revendication 1, caractérisé en ce que dans le cas d'un cuir chevelu (1) présentant des cheveux résiduels relativement courts les racines des cheveux résiduels sur le cuir chevelu (1) déterminent le point de fixation (6) sur lequel l'extrémité de fixation d'un cheveu (5) relativement long est mise en contact avec le cuir chevelu et le cheveu (5) est ensuite fondu sur le cheveu résiduel près du point de fixation (6).

4. Procédé selon la revendication 1, caractérisé en ce que les cheveux étrangers sont réunis en touffes de cheveux étrangers et chaque touffe de cheveux étrangers est comprimée à l'extrémité de fixation et est mise en contact en un point de fixation (6) avec la partie d'un cuir chevelu (1) et en ce que le rayon laser (8) atteint le cuir chevelu (1) au point de fixation (6).

5. Procédé selon la revendication 1, caractérisé en ce que les cheveux étrangers sont amenés en touffes, sont isolés en différents cheveux avant la fonte et en ce que chaque cheveu est ensuite fondu séparément.

6. Procédé selon la revendication 1, caractérisé en ce que le point de fixation (6) est marqué optiquement avant la fonte.

7. Procédé selon la revendication 2, caractérisé en ce que l'orientation du rayon laser (8) détermine le point de fixation (6) du cheveu (5) et en ce que le cheveu (5) est immobilisé lorsque son extrémité de fixation se trouve au point de fixation (6).

8. Procédé selon la revendication 2 ou 3, caractérisé en ce que le positionnement du cheveu (5) par rapport au point de fixation (6) est observé.

9. Procédé selon la revendication 2 ou 3, caractérisé en ce que le rayon laser (8) est guidé de manière mobile et en ce qu'une trajectoire de déplacement du rayon laser (8) sur le cuir chevelu (1) relie une multiplicité de points de fixation (6) les uns avec les autres sur lesquels les différentes extrémités de fixation (6) des cheveux (5) ou des touffes de cheveux étrangers sont immobilisées.

10. Procédé selon la revendication 2, caractérisé en ce qu'une multiplicité de cheveux (5) sont en contact par leurs extrémités de fixation avec le cuir chevelu (1) et sont fondus sur le cuir chevelu (1) les uns après les autres en modifiant la direction du rayon laser (8).

11. Procédé selon la revendication 3, caractérisé en ce qu'une multiplicité de cheveux (5) sont chacun en contact par leurs extrémités de fixation avec le cuir chevelu (1) et un cheveu résiduel correspondant et sont fondus les uns après les autres en modifiant la direction du rayon laser (8), sur le cheveu résiduel dans la zone du point de fixation (6).

12. Dispositif d'exécution du procédé selon la revendication 1, caractérisé en ce qu'une tête d'intervention mobile (2) renferme une source (3) de rayon laser ainsi qu'un dispositif de guidage (4) pour un cheveu étranger (5), de telle sorte que le cheveu étranger (5) comportant une extrémité de fixation puisse être guidé vers l'avant à travers un orifice d'application (7) et qu'un point de fixation (6) du cheveu étranger (5) soit déterminé par le contact de l'extrémité de fixation du cheveu étranger (5) avec une partie du cuir chevelu (1) au point d'intersection avec un rayon laser (8) émis par la source (3) de rayon laser, le cas échéant en coïncidence pratique avec une racine d'un cheveu résiduel sur la partie du cuir chevelu (1).

13. Dispositif selon la revendication 12, caractérisé en ce que la tête d'intervention (2) présente un dispositif optique de marquage (9) comportant un câble optique (10).

14. Dispositif selon la revendication 12, caractérisé en ce que la tête d'intervention (2) présente un dispositif optique d'observation (11), en particulier un microscope.

15. Dispositif selon la revendication 12, caractérisé en ce que le dispositif de guidage (4) comprend un dispositif d'amenée, un dispositif de centrage et un dispositif d'immobilisation.

16. Dispositif selon la revendication 15, caractérisé en ce que le dispositif de guidage (4) comprend des galets transporteurs.

17. Dispositif selon la revendication 16, caractérisé en ce que les galets transporteurs aident à la formation de différents cheveux (5) à partir d'une touffe de cheveux étrangers.

18. Dispositif selon la revendication 15, caractérisé en ce que le dispositif de guidage reçoit des touffes de cheveux étrangers desquelles peuvent être isolés différents cheveux (5) dans le dispositif de centrage.

19. Dispositif selon la revendication 15, caractérisé en ce que le dispositif de guidage aide à la formation de différentes touffes de cheveux étrangers à partir d'une touffe de cheveux étrangers.

20. Dispositif selon la revendication 12, caractérisé en ce qu'une multiplicité de cheveux (5) sont acheminés par le dispositif de guidage (4) vers le lieu d'application et en ce que la source (3) de rayon laser peut effectuer un déplacement relatif par rapport au dispositif de guidage (4).

21. Dispositif selon la revendication 12, caractérisé en ce que la tête d'intervention (2) présente un boîtier on matière plastique dont la paroi de fond est flexible ou porte des corps élastiques d'appui,

22. Dispositif selon la revendication 21, caractérisé en ce que la paroi de fond est maintenue à une distance déterminée de la partie du cuir chevelu (1) par des corps de déplacement, au moins près de l'orifice d'application (7).